# EUROPEAN PATENT SPECIFICATION

(11) **EP 2 653 092 B1**
(45) Date of publication and mention of the grant of the patent: **09.09.2015**
(21) Application number: 11819098.2
(22) Date of filing: 09.12.2011
(51) Int. Cl.: A61B 1/04, A61B 1/00

(54) **GASTROINTESTINAL ELECTRONIC PILL**
GASTROINTESTINALE ELEKTRONISCHE PILLE
PILULE ÉLECTRONIQUE GASTRO-INTESTINALE

(30) Priority: 13.12.2010 ES 201031835
(43) Date of publication of application: 23.10.2013
(73) Proprietor: Medlumics S.L., 28760 Tres Cantos (Madrid) (ES)
(72) Inventor: MARGALLO BALBÁS, Eduardo, E-28008 Madrid (ES); RUBIO GUIVERNAU, José Luis, E-28033 Madrid (ES)
(74) Representative: Carvajal y Urquijo, Isabel
(86) International application number: PCT/ES2011/070845
(87) International publication number: WO 2012/080545

(56) References cited:
- WO-A2-2007/091881
- JP-A- 2004 243 034
- US-A1- 2002 051 512
- US-A1- 2009 177 033
- US-A1- 2009 244 547
- US-B1- 6 485 413
- HERZ PR, CHEN Y, AGUIRRE AD, SCHNEIDER K, HSIUNG P, FUJIMOTO JG, MADDEN K, SCHMITT J, GOODNOW J, PETERSON C: "Micromotor endoscope catheter for in vivo, ultrahigh-resolution optical coherence tomography", OPTICS LETTERS, vol. 29, no. 19, 1 October 2004 (2004-10-01), pages 2261-2263, XP002674630,

## Description

### Field of the Invention

the present invention is included within the field of devices for gastrointestinal tract examination through tomographic methods, preferably by optical coherence tomography.

### Background of the Invention

Traditional colonoscopy causes patients a great deal of soreness and discomfort. Current technology now offers capsules which are capable of passing through the digestive tract acquiring information about it and then sending the information to an external receiver. For example, the Heidelberg capsule is capable of measuring pH levels, while the device described in Patent WO0165995 acquires images of interior tract walls. In both cases, the information is sent wirelessly to an external receiver. However, the abovementioned are lacking in high-resolution sectional information, which is important in the study of tissue histology and, therefore, in providing an accurate diagnosis. Thus, although they allow visual identification and detection of polyps and other at-risk structures, their usefulness is limited as a subsequent biopsy is still required.

This need has been identified repeatedly in literature. U.S. Patent Application 2005/0096526 A1 describes a gastrointestinal capsule with an adjustable image element. Although this application focuses on traditional photographic imaging systems, it raises the possibility of using an alternative imaging element, including means of tomographic information acquisition. However, the application only conveys the need to solve the problem at issue with this invention, and at no time specifies the means for achieving it.

U.S. Patent Application 2007/0142708 A1 mentions the introduction of an ultrasonic oscillator within a gastrointestinal imaging capsule. This technology, alternative in some applications to optical coherence tomography, has significant limitations in view of integration into a gastrointestinal capsule. The ultrasonic oscillator provides inferior spatial resolution, requires a transfer medium to adapt the acoustic impedances and uses bulkier instrumentation. Furthermore, the application does not describe the integration of ultrasound imaging technology in a gastrointestinal capsule.

Patent Application WO 2008/012701 A1 describes a gastrointestinal video capsule with a variable lens system that allows it to direct beams of light from light sources for imaging in combination with an image sensor. This patent application includes an exemplary application of the invention, in which it is used for an optical coherence tomography system insofar as it could possibly be integrated into gastrointestinal capsules in the future. The application recognizes the importance of obtaining tomographic information but does not describe any solutions.

Application JP 2004/243034A describes how to integrate a full-field OCT (Optical Coherence Tomography) system using a Mirau interferometer. By its very nature, it is based on free-space optical components and is incompatible with the use of integrated optics. One of the invention's significant limitations is that the application of a Mirau interferometer like the one described is very difficult in a situation where distances to the object vary, because various opto-mechanical elements must be adjusted in a range comparable to the desired scan; a distance range comparable to the dimensions of the capsule (10-20mm) cannot be achieved in practice, and the document itself acknowledges that the maximum distance covered by the axial scan is 1-2mm. As a result of the system's inability to adjust its scanning range beyond 1-2mm, the invention described in JP 2004-243034 is reduced to creating lateral images of the sample, and therefore may not produce images of the entire intestinal tract. Another of the invention's significant limitations is that the optical image must coincide with the Mirau interferometer's field of view. This second field of view must be small to obtain the microscopic resolution characteristic of OCT technology which provides histological information. However, when imaging for diagnostic purposes, the macroscopic lateral size of the lesion and its environment can be orders of magnitude greater than the interferometer's field of view.

All of these limitations make the implementation of the invention described in JP 2004-243034 very difficult, and they seriously compromise the potential diagnostic value of optical coherence tomography in this application.

Application US 2009/0177033 A1 discloses a capsule for OCT.

Therefore, a gastrointestinal capsule integrating an OCT imaging system that could be adapted to work at different distances from the sample was desirable. A capsule with these characteristics would be able to obtain tomographic information for the entire intestinal tract and, combining the OCT system with video equipment, it could possibly offer doctors not only video information like other available capsules, but also tomographic information for the structures that appear in the video, providing a virtual biopsy that could preclude the need for actual biopsies.

### Description of the Invention

The invention aims to solve both the limitations of the state of the art and the severe discomfort and morbidity that digestive endoscopy procedures cause for patients, minimizing the number of associated biopsy interventions. The procedures that might bring a qualitative advance are esophagogastroduodenoscopy, proctosigmoidoscopy, colonoscopy, endoscopic retrograde cholangiopancreatography or enteroscopy. Especially in the latter cases, the difficulties of standard endoscopic access make the search for alternative diagnostic means particularly advisable.

The invention describes a gastrointestinal capsule that integrates an OCT system capable of adapting its operating range to a wide interval of distances to the object (up to several centimetres) and the lateral scanning range of which can be selected and varied over a wide range of areas within the field of view of the focusing optics, decoupling the OCT scanning area and the general field of view of said optics.

The invention also describes ways to integrate the OCT system with a video system the field of view of which coincides with the general optic but is independent of the area scanned by the OCT system at any time. Methods by which, if desired, the relative position of the two can be recorded are also described. The invention furthermore describes implementation solutions based on integrated optics that demonstrate that the invention is feasible in the compact size that is required in a gastrointestinal capsule. Regarding non-tomographic imaging systems, the present invention offers the advantage of being able to obtain axial information combined with the video image, which is of great diagnostic value, making actual biopsies avoidable.

The invention comprises an outer biocompatible shell capable of withstanding the chemical environment of the digestive system as well as information acquisition means, processing means and electric power supply means in an ultra-compact implementation of a high-resolution tomographic imaging system. The processing means can send the collected information to an external system for study and analysis, although there may also be an internal storage unit included. The capsule can be supplied power through an inductive link or by other means (for example, by battery).

The information acquisition means comprise a tomographic imaging system that uses optical coherence tomography built with planar technology and solid-state components. This system comprises light sources, detectors and integrated optical elements arranged according to one of the many configurations used in optical coherence tomography. In contrast to technology used in the state of the art, the invention does not provide a full-field OCT, but rather a scanning beam OCT. The scanning beam OCT is necessary for using guided optics and, especially, for integrated optics. Using integrated optics offers several advantages: manufacturing cost, system compactness, reliability and durability. Manufacturing cost and system compactness are due to decreasing marginal costs and the high integration density of planar fabrication techniques, while reliability and durability are due to the minimization of moving parts and electromechanical components.

The sampling arm of the optical coherence tomography system is connected to electromechanical means that allow moving the point of intersection of the light beam with the tissue surface to analyse. The capsule allows moving the point of intersection of the light beam with the tissue surface in a wide working range. The beam's angular orientation can be adjusted using MEMS (MicroElectroMechanical System) elements or other electromechanical devices to produce the movement of an optical component in the beam's path. The system would include a set of fixed-focus or, preferably, variable lenses to focus the light beam on the desired tissue area. In the case of fixed-focus lenses, the working range is limited to a distance range pre-established by the optical design. In the case of variable optics, a wide working distance range can be accessed, which is preferable. The high-speed lateral scan particular to OCT can be implemented with the same MEMS components or other electromechanical devices used to guide the beam across the tissue area of interest.

The OCT system will be preferably combined with video equipment based on a solid-state sensor. For this purpose, it is possible to use a beam separation system, according, for example, to the wavelengths of light making up the beam. A possible simple implementation would be based on a dichroic mirror that reflects the wavelength range used by the optical coherence tomography system toward said system and allows another wavelength range to pass to the video equipment. Alternatively, said beam separation system could be based on a partially reflective mirror.

The video equipment will be used to relate the tomographic information to the macroscopic anatomical structures analyzed. Preferably, the OCT system and video equipment will be linked to pinpoint the position of the volume studied by means of the OCT system in the video image. If the wavelength of the beam used by the OCT system can be detected by the CCD/CMOS (Charge Coupled Device/Complementary Metal-Oxide-Semiconductor) sensor of the video equipment (in silicon for wavelengths less than 1000nm), and the beam separation system is designed in such a way that it allows for the partial passage of OCT wavelengths to said sensor, then the OCT beam position could be immediately evident in the image once the light scattered in the OCT scanning area is collected by the optical video system and detected in the image sensor as a brighter area.

Otherwise, if the OCT system uses wavelengths longer than those that can be detected by the video equipment's sensor, or if a strict filtering is chosen for the video equipment and the optical coherence tomography system, or if simply preferred for any reason, a mechanical calibration of the electromechanical means that guide the beam can be performed in such as way that, in combination with the target distance of the fixed-focus or variable lens, it can calculate the spatial range covered by the OCT system with respect to the image acquired by the video equipment.

The sensor and electronics for processing the video image can be included on the same substrate in which the waveguides and other planar optical elements for the optical coherence tomography system are constructed, or they can be included on another separate substrate.

The video system can have appropriate wavelength illumination for the excitation of different fluorescence phenomena in the tissue and can be accompanied by suitable filters that remove said excitation wavelengths. This makes it possible to record the areas of tissue with fluorescence emission in order to provide diagnostic information complementary to the histological information obtained through optical coherence tomography.

To the extent that the system is intended to work free of degradation due to chromatic dispersion of the material used for its implementation, it is necessary to equalize it in both arms (reference and sampling) so that the resulting interference pattern is determined only by the source's bandwidth. The same can be said of the birefringence potentially inherent to the chosen waveguide technology. If this birefringence is not perfectly cancelled out in the manufacturing process, it is necessary to equalize it in both arms.

At the same time, to cover a wide focus depth range, a reference arm that is adjustable in length beyond that required to create the OCT image must be used. Specifically, the OCT image has a typical depth of 1-2mm, while the desired focal distance range is a few centimetres. In the invention, this is achieved by switching the reference and/or sampling arms' internal pathways in the integrated optics system between waveguides of different physical lengths.

However, as mentioned, this poses problems in integrated OCT systems, as the technologies with the most common active elements, such as silicon, tend to be relatively dispersive and birefringent. Consequently, making non-zero the mean difference in length in the material during the scan between reference arm and sampling arm leads to resolution problems in the final system, as the chromatic dispersion between the arms gets unbalanced.

The above problem can be solved in other ways. A first way is by using an active element for dispersion compensation in one of the arms. Said active element can be based on dispersive designs such as photonic crystals or chirped Bragg gratings, but can also make use of waveguide segments which, given the geometric or material characteristics thereof, present an anomalous chromatic dispersion. The design of this active element must pay special attention to the bandwidth on one hand, and the higher orders of chromatic dispersion on the other. By acting on the dispersion coefficients at different orders by changing the material's refractive index through thermo-optical or injection of charge carriers effects, for example, it is possible to adjust the operating point of the active element and compensate for the induced chromatic dispersion. Certainly, these elements also affect the group delay, competing with the physical change in the effective length of the arms, so its design must be properly equalized with the physical lengths to obtain the desired total delay differences.

Another solution is to implement devices in a hybrid technology that combines active materials such as silicon with low-dispersion and/or birefringent materials such as silicon (oxy)nitride or silicon oxide. If the combination of said technologies in a single process turns out to be too complex technically, or undesirable for economic or other reasons, it is possible to use a solution based on two different substrates with an active element and a passive element that includes no more than an array of guides ending in reflectors with different physical lengths. It is also useful to consider coupling an array of discrete fibres with different lengths and ending in a reflector to the substrate on which the optical coherence tomography system is produced.

Additionally, the invention may include means for estimating the position of the capsule in the body in order to facilitate subsequent interpretation of the collected information. The position of the capsule can be estimated using different *in vivo* locating and navigation technologies, including the use of triangulation from an electromagnetic signal transmitted from inside the shell or by generating external magnetic fields detected in the shell.

Another option for estimating the position of the capsule is to include in the device some kind of motion sensor, such as an accelerometer or gyroscope, in order to provide information relative to the movement of the device and, therefore being able, for instance, to begin imaging once device movement is detected.

Consequently, the electronic gastrointestinal capsule protected by an outer biocompatible shell resistant to the environment of the digestive system internally comprises:
- at least one light source;
- splitting means configured to split the light beam coming from the light source and direct it toward the reference arm and sampling arm;
- an adjustable group delay element depending on the distance to the tissue to analyse located in at least one of the arms;
- optical moving means configured to move the intersection of the light beam from the sampling arm across the tissue to analyse;
- an optical system configured to focus the light beam from the sampling arm on the tissue surface to analyse;
- interference means configured to produce interference between the reflected light coming from the reference arm and sampling arm;
- at least one detector receiving said interference between the light coming from the reference arm and sampling arm;
- processing means configured to process the information acquired by the detector;
- power supply means configured to electrically supply power to the capsule without a physical connection to the outside.

The adaptable group delay element can comprise at least one optical switch that chooses one among a plurality of optical paths where said optical paths introduce different pre-established group delays.

Additionally, said adaptive group delay element can interact with the optical system to jointly adjust the group delay and the focusing distance.

Preferably, the capsule will comprise an active element for chromatic dispersion compensation in at least one of the arms, selected from:
- photonic crystals;
- chirped Bragg gratings;
- waveguide segments the characteristics of which cause anomalous chromatic dispersion.

The sampling arm and reference arm may share at least partially the same physical path.

The processing means can comprise a storage unit configured to store the information processed by the processing means.

Preferably, the processing means comprise a communications unit configured to send the information processed by the processing means to an external communications device and to receive instructions from said external communications device.

The optical moving means will preferably comprise electromechanical means configured to move the point of intersection of the light beam with the tissue surface to analyse.

Preferably, the capsule will comprise video equipment configured to capture images of the tissue in the capsule surroundings.

The optical system can comprise a beam separation system according to the wavelength, directing the wavelength range used by the optical coherence tomography system to said system and another wavelength range to the video equipment.

Additionally, the capsule can comprise identifying means configured to locate the position of the outgoing light beam of the sampling arm of the optical coherence tomography system in the image obtained by the video equipment.

The identifying means can interact with the optical moving means and the optical system to orient the position of the outgoing light beam of the sampling arm according to the instructions received by the external communications device.

The video equipment can comprise optical filters according to the wavelength and a complementary light source that allow capturing the fluorescence coming from the tissue in the capsule surroundings.

Preferably, the capsule will include a motion sensor that will provide information relative to the movement of the capsule.

The capsule can comprise a position sensor that provides information about the position of the capsule.

The concept of optical coherence tomography integrated on a substrate is possible by means of integration in planar technology of one of the key OCT elements, such as the rapid scanning variable delay line, using the thermooptic effect of silicon. Because of this integration and the developments particular to this invention, it is possible to design an OCT system integrated within a gastrointestinal capsule. Other implementations of optical coherence tomography, such as the frequency domain, may also be subject to miniaturization by using integrated optics by means of array waveguide gratings. Swept-source based systems using variable frequency solid-state sources can also be integrated according to the developments particular to this invention.

The present invention provides a solution for functionality that has been nonexistent to date, and allows it to obtain tomographic information on potentially neoplastic tissue noninvasively and with minimal discomfort and risk to the patient.

The implementation of the invention would entail some degree of investment by health institutions; however, the reuse of the capsules and the equipment that the former require would enhance the profitability of the necessary investment. Implementation would also require increased human labour dedicated to managing the device due to the high volume of data produced. This volume of data can be controlled with proper planning by using imaging methods to limit operating time and to properly select the regions where data is obtained. Furthermore, it is possible to acquire tomographic data autonomously or semi-autonomously, and to apply filtering algorithms to reduce the amount of information acquired that must be processed manually. If acquisition is automatic, it is possible to associate and subordinate the tomographic information to the video information, so that a doctor can choose the sections to study by observing the video recording.

### Brief Description of the Drawings

A series of drawings which aid in better understanding the invention, expressly relating to an embodiment of said invention presented as an illustrative and non-limiting example thereof, will be briefly described below.
Figure 1 shows a plan view of the electronic gastrointestinal capsule.
Figure 2 shows a section of the electronic gastrointestinal capsule.
Figure 3 shows a section of the electronic gastrointestinal capsule where the inductive link power supply means have been replaced with a battery.
Figure 4 shows the electronic gastrointestinal capsule during use, directing and focusing the light which is used to obtain tomographic images of the tissue to examine.
Figure 5 shows an embodiment of an adaptable delay element in which two switches direct the light of an entrance guide to an exit guide by means of one among a plurality of intermediate guides, each of which intermediate guides is characterized by different fixed delays.
Figure 6 shows the location of the tomographic image on the video image, linking the high-resolution tomographic information to the macroscopic video image.

### Detailed Description of an Embodiment

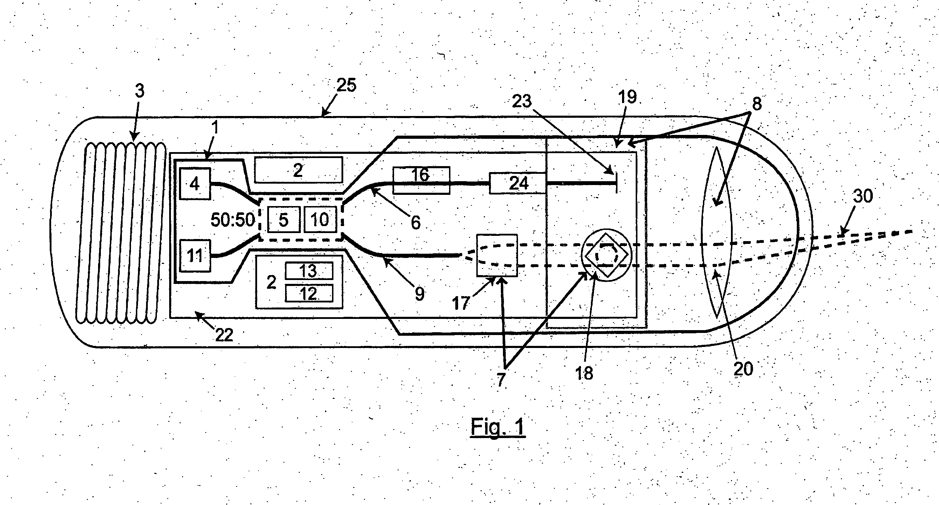

Figures 1, 2 and 3 show views of a capsule the information acquisition means 1 of which, configured to obtain tomographic information about the tissue in the capsule surroundings by transmitting and receiving oscillating disturbances, comprise an optical coherence tomography system contained within the external shell 25 that surrounds the gastrointestinal capsule. This shell 25 must be biocompatible, and part of it must be transparent to the waves used to facilitate transmission and reception by the information acquisition means 1.

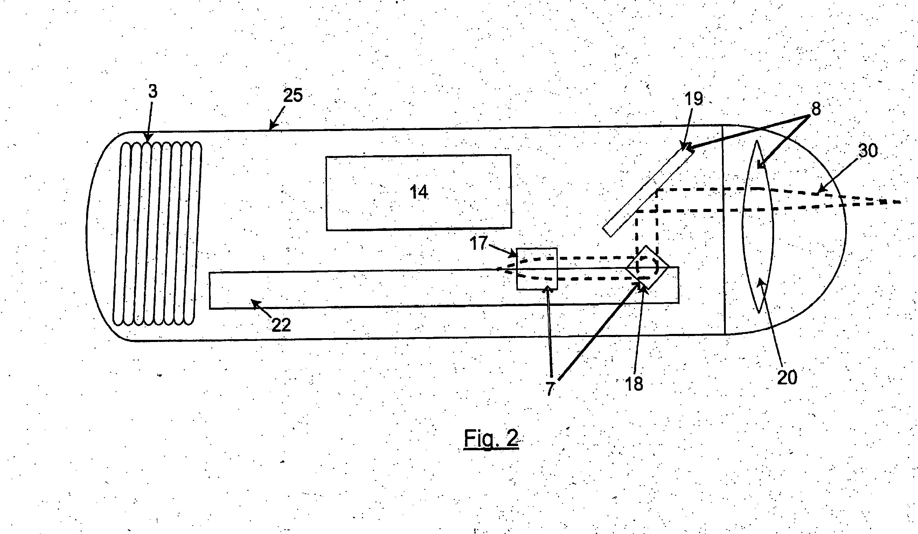

Figures 1 and 2 show power supply means 3 based on a coil the function of which is to electrically supply power to the capsule by producing an induced current from an alternating magnetic field generated externally to the device. The presence of several coil orientations would facilitate the continuity of the electric power supply regardless of the capsule's physical orientation in relation to the field, although it is not strictly necessary. The coils can also be part of the capsule's communications unit 13. In this sense, the external magnetic field can be modulated to transmit information to the capsule, and the link can be made bidirectional if some of the known techniques for this purpose, such as load shift keying, are applied to send information to an external system. Alternatively, the capsule's communications unit can use a transmission and reception channel different from the inductive link.

An optical coherence tomography system implemented on a substrate 22 by means of integrated opticscan also be observed in the figures. The light from a compact light source 4, such as a solid-state source (for example, a superluminescent diode or SLD), is conducted to splitting means 5, in Figure 1 this is a 50/50 coupler, that splits light into two arms, a reference arm 6 and a sampling arm 9.

The reference arm 6 includes, in this basic implementation in the time domain, an ultra-compact thermooptic delay line 16 that provides the axial scan. Other OCT system configurations would be possible as it is well known (frequency domain, swept-source, etc.). An adaptable group delay element 24 is also shown in the reference arm 6 that can adjust the interferometer for a wide set of distances in the sampling arm 9, corresponding to the different distances to the tissue to examine. At the end of the reference arm 6 there is a reflector 23 that sends the light back through said arm 6.

Light from the sampling arm 9 is sent to optical moving means 7 that serve to move the intersection of the light beam 30 coming from the sampling arm 9 with the tissue to analyse. In the system shown in the figures, the optical moving means 7 consist of means to collimate the light exiting the sampling arm 9, for example, some kind of compact lens such as those based on a gradient index 17 (GRIN), which direct the collimated beam towards a moving mirror 18, as shown in the figure with an implementation using MEMS microfabrication techniques. Said moving mirror 18, in addition to orienting the beam 30 on the point of interest in the tissue, can also provide the side scan that complements the axial scan for tomographic imaging. The light reflected in the moving mirror 18 is then directed to an optical system 8, which aims to orient and focus the beam 30 on the tissue. In the system shown in the figures, the optical system 8 consists of a beam separation system 19, such as a dichroic mirror or a semi-reflective mirror for example, which receives the collimated beam coming from the moving mirror 18 and which in turn reflects the beam towards a lens 20, which focuses it on the tissue to study. Using the dichroic mirror is intended to direct the wavelength range detected by the video equipment 14 towards said equipment and another wavelength range towards the optical coherence tomography system, thus allowing both to share the optical system 8 without loss of power. A semi-reflective mirror achieves the same effect, but optical power is lost. The lens 20 (or lens system) will preferably be variable focus to offer a wide working distance range. This capacity can be provided by means of moving optical elements or by means of lenses with focal length that can be adjusted by other means, such as liquid crystals, electrowetting, etc.

Focus adjustment would preferably be performed automatically and in combination with the delay adjustment introduced by the adaptable group delay element 24 in the reference arm 6, as both relate to the distance to the tissue to analyse. Recording these distances for different angular directions results in the construction of the tissue surface topography, which can also be transmitted to the outside and presented to the user as an element with potential diagnostic value.

The reflected light coming from the tissue is collected again by this lens 20 and directed back towards the sampling arm 9 in the integrated optics substrate 22, reversing the path previously taken. The two light components coming from the reference arm 6 and the sampling arm 9, respectively, are combined by interference means 10, which in this implementation coincide with the splitting means (5) and the resulting signal is detected at the detector 11. If the technological manufacturing processes so require, the electronic processing means 2, which include a storage unit 12 and a communications unit 13, can be implemented in the same substrate 22 used for the integrated optics, as illustrated in the figures, or it can be produced in a separate substrate. Figure 2 shows a side view in which the position of the video equipment 14, placed behind the beam separation system 19, can be seen. The video equipment 14 includes tissue lighting means in the visible spectrum to form a suitable image on the sensor. The image sensor can in principle also be part of the substrate 22 in which the waveguides and other planar optical elements of the optical coherence tomography system are produced, especially if the manufacturing technology is compatible with the integrated electronics production processes, with a positive impact on the capsule's final size.

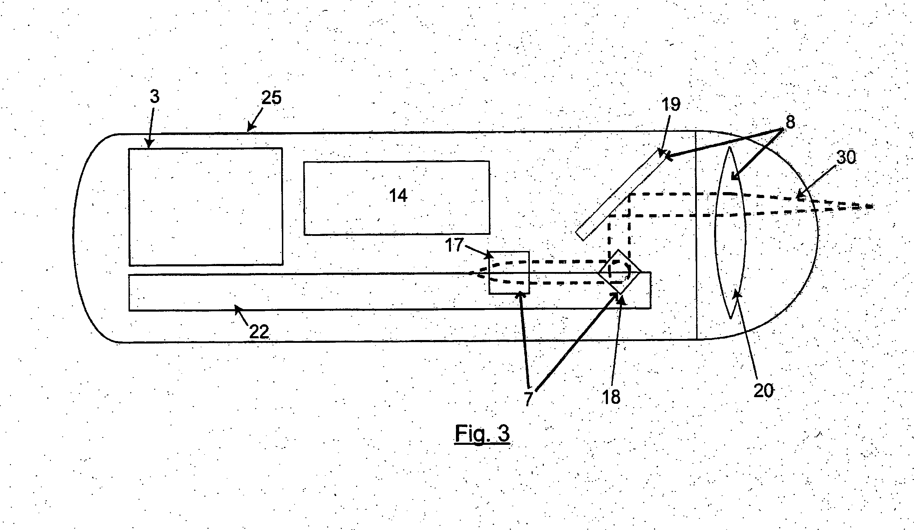

Figure 3 replaces inductive link coils with a battery to implement the capsule's power supply means 3. The communications unit 13 would not be employed in this implementation through the inductive link, but would need an alternative route, such as a radio frequency link.

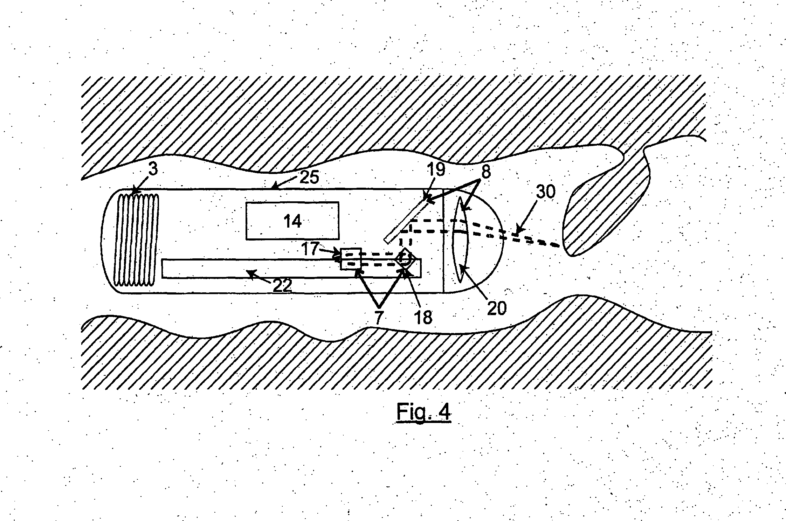

Figure 4 shows the capsule during use in a point of the digestive tract where there is tissue of interest to analyse. The capsule orients and focuses the OCT beam on said area, producing high-resolution tomographic images.

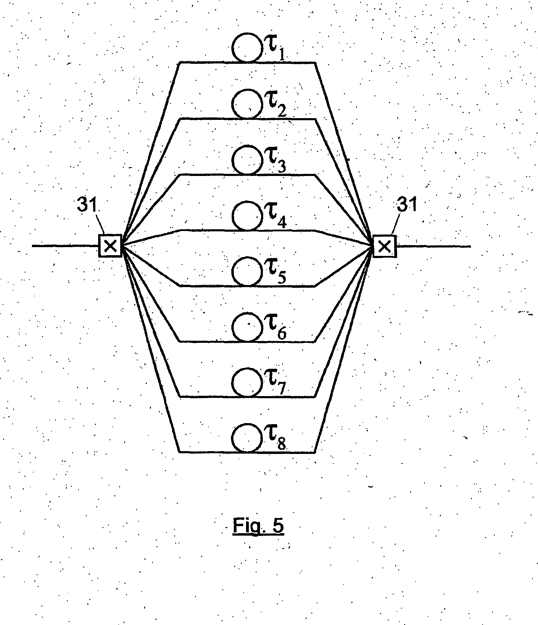

Figure 5 shows an embodiment of an adaptable delay element based on optical switches 31, preferably implemented using integrated optics. These optical switches 31 can be established using multimode interference devices (MMI), or a cascade of actionable directional couplers. The figure shows two switching elements capable of jointly directing light from an entrance guide towards an intermediate guide among several guides, characterized by fixed and different group delays, and in turn, from said intermediate guide to an exit guide symmetrically. Waveguide segments with different group delays can be obtained, for example, by means of waveguides of different lengths.

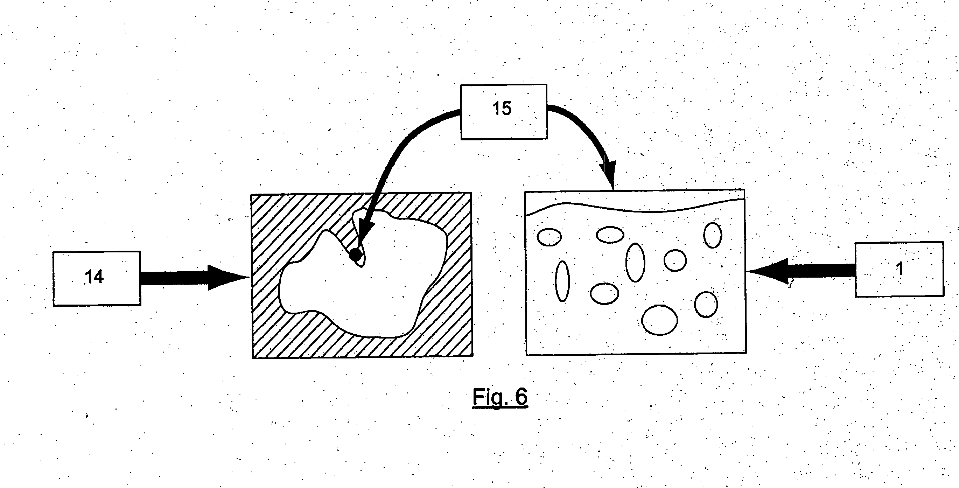

Figure 6 shows the process of locating the scanned area on the video image. This process provides a link between the high-resolution tomographic image and the macroscopic video image. To establish this link, the capsule must have identifying means 15 for identifying the position of the light beam used for OCT with coordinates from the video image. Therefore, the position of the beam can be indicated on the video image at all times. As previously mentioned, the identifying means 15 may consist of a calibration of the electromechanical elements that move the beam in the optical moving means 7 and of the optical system 8, in position sensors of the optical elements responsible for scanning the light beam, or in the direct detection of the beam position on the image sensor.

Having clearly described the invention, it is noted that the particular embodiments described above are susceptible to changes provided they do not alter the fundamental principle of the invention.

## Claims

1. An electronic gastrointestinal capsule being protected by an outer biocompatible shell resistant to the environment in the digestive system and by internally comprising:
- at least one light source (4);
- splitting means (5) configured to split the light beam coming from the light source (4) and direct it to the reference arm (6) and the sampling arm (9);
- an adjustable group delay element (24) depending on the distance to the tissue to analyse, located in at least one of the arms (6,9);
- optical moving means (7) configured to move the intersection of the light beam (30) of the sampling arm (9) across the tissue to analyse;
- an optical system (8) configured to focus the light beam (30) from the sampling arm (9) on the tissue surface to analyse;
- interference means (10) configured to produce interference between the reflected light coming from the sampling arm (9) and reference arm (6);
- at least one detector (11) receiving said interference between the light coming from the reference arm (6) and sampling arm (9);
- processing means (2) configured to process the information acquired by the detector (11);
- power supply means (3) configured to electrically supply power to the capsule without a physical connection to the outside;
- video equipment (14) configured to capture images of tissue in the capsule surroundings; and
- identifying means (15) configured to identify the position of an outgoing light beam from the sampling arm (9) of an optical coherence tomography system in an image obtained by the video equipment (14).

2. The electronic gastrointestinal capsule according to claim 1, **characterised in that** the adjustable group delay element (24) comprises at least one optical switch (31) that chooses one among a plurality of optical paths where said optical paths introduce different pre-established group delays.

3. The electronic gastrointestinal capsule according to any of the preceding claims, **characterised in that** the adjustable group delay element (24) interacts with the optical system (8) to jointly adjust the group delay and the focusing distance.

4. The electronic gastrointestinal capsule according to any of the preceding claims, **characterised in that** it comprises an active element for chromatic dispersion compensation in at least one of the arms (6,9), selected from:
- photonic crystals
- chirped Bragg gratings
- waveguide segments the characteristics of which cause anomalous chromatic dispersion.

5. The electronic gastrointestinal capsule according to any of the preceding claims, **characterised in that** the sampling arm (9) and reference arm (6) share at least partially the same physical path.

6. The electronic gastrointestinal capsule according to any of the preceding claims, **characterised in that** the processing means (2) comprise a storage unit (12) configured to store the information processed by the processing means (2).

7. The electronic gastrointestinal capsule according to any of the preceding claims, **characterised in that** the processing means (2) comprise a communications unit (13) configured to send information processed by the processing means (2) to an external communications device and to receive instructions from said external communications device.

8. The electronic gastrointestinal capsule according to any of the preceding claims, **characterised in that** the optical moving means (7) comprise electromechanical means configured to move the point of intersection of the light beam (30) with the tissue surface to analyse.

9. The electronic gastrointestinal capsule according to claim 1, **characterised in that** the optical system (8) comprises a beam separation system (19) according to the wavelength, directing the wavelength range used by the optical coherence tomography system to said system and another wavelength range to the video equipment (14).

10. The electronic gastrointestinal capsule according to claim 1, **characterised in that** the identifying means (15) interact with the optical moving means (7) and the optical system (8) to orient the position of the outgoing light beam of the sampling arm (9) according to the instructions received by the external communications unit.

11. The electronic gastrointestinal capsule according to any of claims 9 or 10, **characterised in that** the video equipment (14) comprises optical filters according to the wavelength and a complementary light source that allow capturing fluorescence coming from the tissue in the capsule surroundings.

12. The electronic gastrointestinal capsule according to any of the preceding claims, **characterised in that** it comprises a movement sensor providing information relative to the movement of the capsule.

13. The electronic gastrointestinal capsule according to any of the preceding claims, **characterised in that** it comprises a position sensor providing information relative to the position of the capsule.

## Patentansprüche

1. Elektronische gastrointestinale Kapsel, durch eine äußere biokompatible Hülle geschützt, die gegen das Milieu im Verdauungssystem beständig ist, und intern umfassend:
- mindestens eine Lichtquelle (4);
- Spaltmittel (5), die zum Spalten des von der Lichtquelle (4) kommenden Lichtstrahles und um diesen an den Referenzarm (6) und den Probennahmearm (9) zu richten, ausgebildet sind;
- ein in Abhängigkeit des Abstandes zu dem zu analysierenden Gewebe einstellbares Gruppenlaufzeitelement (24), das in zumindest einer der Arme (6, 9) angeordnet ist;
- optische Bewegungsmittel (7), die zum Bewegen des Schnittpunktes des Lichtstrahles (30) des Probennahmearmes (9) über das zu analysierende Gewebe ausgebildet sind;
- optisches System (8), das zum Fokussieren des Lichtstrahles (30) von dem Probennahmearm (9) auf die Oberfläche des zu analysierenden Gewebes ausgebildet ist;
- Interferenzmittel (10), die zum Erzeugen einer Interferenz zwischen dem von dem Probennahmearm (9) kommenden reflektierten Licht (9) und dem Referenzarm (6) ausgebildet sind;
- mindestens ein Detektor (11), der besagte Interferenz zwischen dem von dem Referenzarm (6) kommenden Licht und dem Probennahmearm (9) empfängt;
- Verarbeitungsmittel (2), die zum Verarbeiten der von dem Detektor (11) erfassten Informationen ausgebildet sind;
- Stromversorgungsmittel (3), die für die elektrische Stromversorgung der Kapsel ohne eine körperliche Verbindung zur Außenseite hin ausgebildet sind;
- Videoausrüstung (14), die zum Erfassen von Bildern des Gewebes in der Umgebung der Kapsel ausgebildet ist; und
- Identifizierungsmittel (15), die zum Identifizieren der Position eines von dem Probennahmearm (9) eines optischen Kohärenztomographie-Systems abgehenden Lichtstrahles in einem von der Videoausrüstung (14) erhaltenen Bild, ausgebildet sind.

2. Elektronische gastrointestinale Kapsel nach Anspruch 1, **dadurch gekennzeichnet, dass** das einstellbare Gruppenlaufzeitelement (24) mindestens einen optischen Schalter (31) umfasst, der einen von mehreren optischen Pfaden auswählt, wobei diese optische Pfade verschiedene vorgegebene Gruppenlaufzeiten einführen.

3. Elektronische gastrointestinale Kapsel nach einem der vorhergehenden Ansprüche, **dadurch gekennzeichnet, dass** das einstellbare Gruppenlaufzeitelement (24) mit dem optischen System (8) zusammenwirkt, um gemeinsam die Gruppenlaufzeit und die Fokussierungsdistanz einzustellen.

4. Elektronische gastrointestinale Kapsel nach einem der vorhergehenden Ansprüche, **dadurch gekennzeichnet, dass** sie ein aktives Element zur Kompensation der chromatischen Dispersion in zumindest einer der Arme (6, 9) umfasst, ausgewählt aus:
- photonischen Kristallen
- gechirpten Bragg-Gittern
- Wellenleitersegmenten, dessen Merkmale anormale chromatische Dispersion verursachen.

5. Elektronische gastrointestinale Kapsel nach einem der vorhergehenden Ansprüche, **dadurch gekennzeichnet, dass** der Probennahmearm (9) und der Referenzarm (6) sich zumindest teilweise den gleichen körperlichen Pfad teilen.

6. Elektronische gastrointestinale Kapsel nach einem der vorhergehenden Ansprüche, **dadurch gekennzeichnet, dass** die Verarbeitungsmittel (2) eine Speichereinheit (12), die zum Speichern der durch die Verarbeitungsmittel (2) verarbeiteten Informationen ausgebildet ist, umfassen.

7. Elektronische gastrointestinale Kapsel nach einem der vorhergehenden Ansprüche, **dadurch gekennzeichnet, dass** die Verarbeitungsmittel (2) eine Kommunikationseinheit (13) umfassen, die zum Senden von Informationen, die durch die Verarbeitungsmittel (2) verarbeitet werden, an eine externe Kommunikationsvorrichtung und zum Empfangen von Anweisungen von besagter externen Kommunikationsvorrichtung, ausgebildet ist.

8. Elektronische gastrointestinale Kapsel nach einem der vorhergehenden Ansprüche, **dadurch gekennzeichnet, dass** die optische Bewegungsmittel (7) elektromechanische Mittel umfassen, die zum Bewegen des Schnittpunktes des Lichtstrahles (30) mit der Oberfläche des zu analysierenden Gewebe ausgebildet sind.

9. Elektronische gastrointestinale Kapsel nach Anspruch 1, **dadurch gekennzeichnet, dass** das optische System (8) ein Strahltrennungssystem (19) gemäß der Wellenlänge umfasst, wobei der vom optischen Kohärenztomographie-System benutzte Wellenlängenbereich zu besagtem System und ein anderer Wellenlängenbereich zu der Videoausrüstung (14) geführt wird.

10. Elektronische gastrointestinale Kapsel nach Anspruch 1, **dadurch gekennzeichnet, dass** die Identifizierungsmittel (15) mit den optischen Bewegungsmitteln (7) und dem optischen System (8) zusammenwirken, um die Lage des von dem Probennahmearm (9) abgehenden Lichtstrahles, gemäß der von der externen Kommunikationseinheit empfangenen Anweisungen, zu orientieren.

11. Elektronische gastrointestinale Kapsel nach einem der Ansprüche 9 oder 10, **dadurch gekennzeichnet, dass** die Videoausrüstung (14) optische Filter gemäß der Wellenlänge und eine zusätzliche Lichtquelle, die die Erfassung der Fluoreszenz aus dem Gewebe in der Umgebung der Kapsel ermöglicht, aufweist.

12. Elektronische gastrointestinale Kapsel nach einem der vorhergehenden Ansprüche, **dadurch gekennzeichnet, dass** sie einen Bewegungssensor, der Informationen bezüglich der Bewegung der Kapsel bereitstellt, umfasst.

13. Elektronische gastrointestinale Kapsel nach einem der vorhergehenden Ansprüche, **dadurch gekennzeichnet, dass** sie einen Positionssensor, der Informationen bezüglich der Position der Kapsel bereitstellt, umfasst.

## Revendications

1. Une capsule électronique gastro-intestinale étant protégée par une coquille biocompatible externe résistante à l'environnement au sein du système digestif et comprenant à l'intérieur :
- au moins une source de lumière (4) ;
- des moyens de division (5) configurés pour diviser le faisceau lumineux provenant de la source de lumière (4) et pour le diriger vers le bras de référence (6) et le bras d'échantillonnage (9) ;
- un élément de retard adaptable (24) qui dépend de la distance du tissu à analyser, situé dans au moins l'un des bras (6,9) ;
- des moyens de déplacement optique (7) configurés pour déplacer l'intersection du faisceau lumineux (30) du bras d'échantillonnage (9) à travers le tissu à analyser ;
- un système optique (8) configuré pour centrer le faisceau lumineux (30) provenant du bras d'échantillonnage (9) sur la surface du tissu à analyser ;
- des moyens d'interférence (10) configurés pour produire de l'interférence entre la lumière réfléchie provenant du bras d'échantillonnage (9) et le bras de référence (6) ;
- au moins un détecteur (11) qui reçoit ladite interférence entre la lumière provenant du bras de référence (6) et le bras d'échantillonnage (9) ;
- des moyens de traitement (2) configurés pour traiter les informations obtenues par le détecteur (11) ;
- des moyens d'alimentation en énergie (3) configurés pour alimenter la capsule en énergie électrique sans une connexion physique à l'extérieure ;
- équipement vidéo (14) pour capturer des images de tissu autour de la capsule ; et
- des moyens d'identification (15) configurés pour identifier la position d'un faisceau lumineux sortant, provenant du bras d'échantillonnage (9) d'un système de tomographie par cohérence optique dans une image obtenue par l'équipement vidéo (14).

2. La capsule électronique gastro-intestinale selon la revendication 1, **caractérisée en ce que** l'élément de retard de groupe adaptable (24) comprend au moins un commutateur optique (31) qui choisit un parmi une pluralité de trajets optiques où lesdits trajets optiques introduisent différents retards de groupe préétablis.

3. La capsule électronique gastro-intestinale selon l'une quelconque des revendications précédentes, **caractérisée en ce que** l'élément de retard de groupe adaptable (24) interagit avec le système optique (8) pour adapter conjointement le retard de groupe et la distance de mise au point.

4. La capsule électronique gastro-intestinale selon l'une quelconque des revendications précédentes, **caractérisée en ce qu'**elle comprend un élément actif pour la compensation de dispersion chromatique dans au moins l'un des bras (6,9), choisis parmi :
- des cristaux photoniques ;
- des réseaux Bragg à pas variables,
- des segments de guide d'ondes dont les caractéristiques provoquent une dispersion chromatique anomale.

5. La capsule électronique gastro-intestinale selon l'une quelconque des revendications précédentes, **caractérisée en ce que** le bras d'échantillonnage (9) et le bras de référence (6) partagent au moins partiellement le même trajet physique.

6. La capsule électronique gastro-intestinale selon l'une quelconque des revendications précédentes, **caractérisée en ce que** les moyens de traitement (2) comprennent une unité de stockage (12) configurée pour stocker les informations traitées par les moyens de traitement (2).

7. La capsule électronique gastro-intestinale selon l'une quelconque des revendications précédentes, **caractérisée en ce que** les moyens de traitement (2) comprennent une unité de communications (13) configurée pour envoyer les informations traitées par les moyens de traitement (2) à un dispositif de communications externes et pour recevoir les instructions provenant dudit dispositif de communications externes.

8. La capsule électronique gastro-intestinale selon l'une quelconque des revendications précédentes, **caractérisée en ce que** les moyens de déplacement optique (7) comprennent des moyens électromécaniques configurés pour déplacer le point d'intersection du faisceau lumineux (30) avec la surface du tissu à analyser.

9. La capsule électronique gastro-intestinale selon la revendication 1, **caractérisée en ce que** le système optique (8) comprend un système de séparation de faisceau (19) selon la longueur d'ondes, dirigeant la plage de longueur d'ondes utilisée par le système de tomographie par cohérence optique vers ledit système et une autre plage de longueur d'onde vers un équipement vidéo (14).

10. La capsule électronique gastro-intestinale selon la revendication 1, **caractérisée en ce que** les moyens d'identification (15) interagissent avec les moyens de déplacement optique (7) et le système optique (8) pour orienter la position du faisceau lumineux sortant du bras d'échantillonnage (9) selon les instructions reçues par l'unité de communications externes.

11. La capsule électronique gastro-intestinale selon l'une quelconque des revendications 9 ou 10, **caractérisée en ce que** l'équipement vidéo (14) comprend des filtres optiques selon la longueur d'ondes et une source de lumière complémentaire qui permet de capturer la fluorescence provenant du tissu autour de la capsule.

12. La capsule électronique gastro-intestinale selon l'une quelconque des revendications précédentes, **caractérisée en ce qu'**elle comprend un détecteur de mouvement fournissant des informations relatives au mouvement de la capsule.

13. La capsule électronique gastro-intestinale selon l'une quelconque des revendications précédentes, **caractérisée en ce qu'**elle comprend un détecteur de position fournissant des informations relatives à la position de la capsule.
